(19) Europäisches Patentamt
European Patent Office
Office européen des brevets

(11) **EP 1 935 384 A1**

(12) **EUROPÄISCHE PATENTANMELDUNG**

(43) Veröffentlichungstag:
**25.06.2008 Patentblatt 2008/26**

(51) Int Cl.:
***A61F 9/01*** *(2006.01)*

(21) Anmeldenummer: **06026333.2**

(22) Anmeldetag: **19.12.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(71) Anmelder: **Schwind eye-tech-solutions GmbH & Co. KG**
**63801 Kleinostheim (DE)**

(72) Erfinder:
• **Arba-Mosquera, Samuel**
**63739 Aschaffenburg (DE)**

• **Klinner, Thomas**
**63743 Aschaffenburg (DE)**

(74) Vertreter: **Patentanwälte**
**Hofstetter, Schurack & Skora**
**Balanstrasse 57**
**81541 München (DE)**

Bemerkungen:
Geänderte Patentansprüche gemäss Regel 137(2) EPÜ.

(54) **Verfahren zum Steuern des Auftreffortes von Laserpulsen bei einer Ablation von Hornhaut an einem Auge**

(57) Beim Ablatieren wird der Mittelpunkt (a) des Limbus und der Mittelpunkt der Pupille gemessen. Aufgrund einer zuvor anhand der Mittelpunkte des scotopischen Auges und des photopischen Auges durch Interpolation ermittelten Beziehung ist ermittelbar, wie der Mittelpunkt der Pupille, gesehen relativ zum Mittelpunkt des Limbus, von dem Zustand bei unverdrehtem Auge abweicht. Es kann eine Winkelgröße (Θ) abgeleitet werden, die die Verdrehung kennzeichnet. Es wird davon ausgegangen, dass der Mittelpunkt des scotopischen Auges, der als Ablationsmittelpunkt dient, genauso verdreht ist, so dass beim Zielen auf relativ zu dem Ablationsmittelpunkt definierte Auftrefforte diese Verdrehung rechnerisch einbezogen werden kann. Das Messen der Position des Mittelpunkts der Pupille ist relativ schnell durchführbar und ermöglicht im Vergleich zum Stand der Technik, bei dem die Verdrehung anhand von Bildern der Iris oder von Adern im Auge ermittelt wurde, eine präzisere Ablation.

FIG. 2

EP 1 935 384 A1

**Beschreibung**

Die Erfindung betrifft ein Verfahren zum Steuern des Auftreffortes von Laserpulsen bei einer Ablation von Hornhaut an einem Auge, nämlich durch geeignetes Steuern einer Ablationsvorrichtung, insbesondere einer Zieleinrichtung derselben.

Die Ablation von Hornhaut an einem Auge dient dazu, in die Hornhaut ein Profil einzuschreiben, das der Hornhaut die Funktion einer Linse verleiht. Auf diese Weise können Fehlsichtigkeiten dauerhaft korrigiert werden. Das Profil entsteht dadurch, dass eine Vielzahl von Laserpulsen abgegeben wird (üblicherweise ca. 20.000 Laserpulse), denen jeweils ein Auftreffort zugeordnet wird. Die Laserpulse verdampfen Hornhaut an dem Auftreffort, so dass jeder Laserpuls etwas Material abträgt (ablatiert). An Auftrefforten, denen viele Laserpulse zugeordnet sind, wird mehr Material ablatiert als an Auftreffpunkten, denen weniger Laserpulse zugeordnet sind. Durch das Festlegen der Koordinaten des Auftreffortes für eine Vielzahl von Laserpulse wird ein Profil definiert. Es werden jeweils zwei Koordinaten des Soll-Auftrefforts relativ zu einem Punkt auf dem Auge festgelegt. Als Ablationszentrum wird üblicherweise der Mittelpunkt der Pupille im Zustand ihrer maximalen Öffnung (bei Dunkelheit), der so genannten scotopischen Pupille, verwendet, denn das in die Hornhaut eingeschriebene Linsenprofil muss unmittelbar vor der Pupille wirken und sollte sich somit an der maximal geöffneten Pupille orientieren. Das Ablationszentrum kann jedoch auch der so genannte Corneal Vertex (Ort der höchsten Erhebung der Hornhaut) sein oder der Mittelpunkt einer definiert beleuchteten Pupille.

Es geht nun darum, die Laserpulse tatsächlich auf den Soll-Auftreffort zu lenken. Hierbei tritt das Problem auf, dass die Pupille bei der Laserbehandlung üblicherweise beleuchtet ist, so dass der Mittelpunkt der scotopischen Pupille während der Behandlung nicht ermittelt werden kann. Der Mittelpunkt der beleuchteten Pupille, also der nicht-scotopischen Pupille, ist üblicherweise nicht mit dem Mittelpunkt der scotopischen Pupille identisch. Gut bei der Laserbehandlung zu erfassen ist jedoch der Limbus des Auges und insbesondere sein Mittelpunkt. Der Limbus (lateinisch für Saum) bezeichnet die Grenzlinie zwischen der Hornhaut des Auges (Cornea) und der Lederhaut des Auges (Sclera), die das Weiße des Auges bildet. Der Limbus ist üblicherweise nährungsweise kreisförmig, so dass ein Mittelpunkt ermittelbar ist.

Um die Laserpulse auf den richtigen Auftreffort lenken zu können, wird vorab die räumliche Beziehung zwischen dem Ablationszentrum (der scotopischen Pupille) und dem Limbus ermittelt, insbesondere werden die beiden Koordinaten, die den Abstand der Mittelpunkte zur scotopischen Pupille und Limbus angeben, gemessen (Zentrumskoordinaten). Die Auftreffortskoordinaten, die relativ zum Mittelpunkt der scotopischen Pupille angegeben sind, können dann unter Verwendung dieser Eichkoordinaten auf den Mittelpunkt des Limbus transformiert werden. Dadurch sind die Positionskoordinaten der Soll-Auftrefforte relativ zum Mittelpunkt des Limbus bekannt. Nun werden während der Behandlung vermittels eines Eyetrackers die Koordinaten des Mittelpunkts des Limbus relativ zu einem absoluten Bezugssystem gemessen, nämlich dem Bezugssystem einer Zieleinrichtung zum Lenken der Laserpulse auf unterschiedliche Orte des Auges. Eine solche Zieleinrichtung umfasst typischerweise mindestens einen Umlenkspiegel. Um den Auftreffort des Laserpulses in zwei Koordinaten zu definieren, können beispielsweise zwei hintereinander angeordnete Umlenkspiegel, jeweils einer für jede Koordinate, bereitgestellt werden, die in Abhängigkeit von diesen Koordinaten entsprechend verschwenkt werden und so Laserpulse zielgenau auf vorbestimmte Auftrefforte an dem Auge lenken. Die auf den Mittelpunkt des Limbus transformierten Koordinaten können in Kenntnis der Absolutkoordinaten des Mittelpunkts des Limbus relativ zur Zieleinrichtung dann auf die Zieleinrichtung transformiert werden, und die Laserpulse können tatsächlich so gelenkt werden, dass sie im Idealfall auf dem Soll-Auftreffort, der ja relativ zum Mittelpunkt der scotopischen Pupille definiert ist, auftreffen, so dass insgesamt das gewünschte Ablationsprofil in die Hornhaut eingeschrieben wird.

Bei der bisher beschriebenen Vorgehensweise ist nicht berücksichtigt, dass sich das Auge während der Behandlung bewegen und verdrehen kann. Um den Effekt einer Verdrehung rechnerisch einzubeziehen und korrigieren zu können, werden bisher Bilder der Iris oder auch der die Iris umgebenden Adern des Auges aufgenommen, und mithilfe von Bildverarbeitungsalgorithmen wird das Ausmaß einer Verdrehung des Auges erfasst, und es erfolgt eine entsprechende Korrektur bei dem Ansteuern der Zieleinrichtung.

Die Verwendung eines Bilderkennungssystems hat den Nachteil, dass dieses relativ langsam arbeitet. So sind üblicherweise kaum mehr als zehn Messungen pro Sekunde heutzutage möglich. Demgegenüber steht eine wesentlich höhere Frequenz, z. B. von 200 Hz, mit der die Laserpulse abgegeben werden. Dies bedeutet, dass erst nach einer Mehrzahl von Laserpulsen, also z. B. zwanzig, eine Korrektur einer möglichen Verdrehung erfolgt. Dadurch stimmen die Ist-Auftrefforte auf der Hornhaut besonders schlecht mit den Soll-Auftrefforten überein.

Es ist Aufgabe der Erfindung, ein Verfahren zum Steuern des Auftreffortes von Laserpulsen bei einer Ablation von Hornhaut an einem Auge anzugeben, bei dem eine mögliche Verdrehung des Auges möglichst schnell korrigiert wird, so dass die Ist-Auftrefforte auf den Hornhaut präziser mit den Soll-Auftrefforten übereinstimmen.

Diese Aufgabe wird durch ein Verfahren nach Patentanspruch 1 gelöst.

Das erfindungsgemäße Verfahren umfasst somit die Schritte:

a) Festlegen oder Messen von zwei Zentrumskoordinaten, die den Abstand eines Ablationszentrums des Auges relativ zum Mittelpunkt des Limbus des Auges bei unverdrehtem Auge angeben,

b) Ermitteln einer Beziehung zwischen dem Durchmesser der Pupille und dem durch zwei Koordinaten angegebenen Abstand des Mittelpunkts der Pupille relativ zum Mittelpunkt des Limbus bei unverdrehtem Auge,

c) Festlegen von zwei Auftrefffortskoordinaten des Soll-Auftreffortes für eine Vielzahl von Laserpulsen relativ zum Ablationszentrum (z. B. dem Mittelpunkt der scotopischen Pupille),

d) Messen des Durchmessers der Pupille bei für die Behandlung (Ablation) vorgegebener Beleuchtung und Zuordnen zweier Koordinaten aufgrund der in Schritt b) ermittelten Beziehung als Referenzkoordinaten, sowie Wiederholen folgender Vorgänge bei unveränderter Beleuchtung:

e) Messen der zwei Ist-Koordinaten, die den Abstand des Mittelpunkts der Pupille zum Mittelpunkt des Limbus angeben,

f) Berechnen zweier auf den Mittelpunkt des Limbus bezogener Positionskoordinaten anhand von:

- jeweils eines ausgewählten Paares von Auftrefffortskoordinaten,
- den beiden Zentrumskoordinaten,
- den zuletzt gemessenen beiden Ist-Koordinaten, und
- den ermittelten beiden Referenzkoordinaten,

g) Messen der Position des Mittelpunkts des Limbus relativ zu einem Bezugssystem einer Zieleinrichtung für Laserpulse,

h) Umrechnen der auf den Mittelpunkt des Limbus bezogenen Positionskoordinaten auf das Bezugssystem der Zieleinrichtung,

i) Einstellen der Zieleinrichtung derart, dass abgegebene Laserpulse zu dem durch die (umgerechneten) Positionskoordinaten angegebenen Ort geleitet werden.

Naturgemäß gehört es zu dem Verfahren, dass die Wiederholungen der einzelnen Vorgänge f) bis i) jeweils mit der Abgabe mindestens eines Laserpulses einhergehen. Es können auch gleich mehrere Laserpulse in Folge abgegeben werden, zum Beispiel mit einer typischen Frequenz von 200 Hz. Hierbei sei darauf hingewiesen, dass im Schritt c) dieselben Auftrefffortskoordinaten für mehrere Laserpulse festgelegt sein können (je nach Abtragetiefe an der Hornhaut). Insgesamt sollte im Rahmen der Wiederholung von Schritt f) jedes Paar von Auftrefffortskoordinaten genauso oft ausgewählt werden, wie es in Schritt c) zu Laserpulsen festgelegt wurde, wobei bekannte Verfahren des Sortierens verwendet werden. Es ist möglich, dass auch bei unverändertem Auftreffort die Schritte f) bis i) neu durchgeführt werden müssen, wenn sich etwas an der Augenstellung verändert hat. In diesem Zusammenhang sei darauf hingewiesen, dass nicht erforderlich ist, dass die Vorgänge e) bis i) als Schritte in vorgegebener Reihenfolge hintereinander durchgeführt werden. Vielmehr handelt es sich bei den Vorgängen e), f) bis h) und i) jeweils um Vorgänge, die mit unterschiedlicher Taktung erfolgen können. In Vorgang f) ist ausdrücklich von den zuletzt gemessenen beiden Ist-Koordinaten die Rede, um zu verdeutlichen, dass in Abwesenheit einer neu gemessenen Ist-Koordinate für einen neuen Auftreffort der Vorgang f) auch dann wiederholt werden kann, wenn der Vorgang e) nicht wiederholt wurde.

Allerdings liegt gerade in der Frequenz, mit der die Ist-Koordinaten aktualisiert werden können, der Vorteil der beanspruchten Erfindung. Der Mittelpunkt der Pupille ist mittels eines geeigneten Eyetrackers mit einer deutlich höheren Frequenz erfassbar als das Bilderkennungssystem im Stand der Technik arbeitet. Somit gehen durch die Verdrehung entstehende Korrekturen besonders schnell in die Berechnung (Schritt f)) ein, und die Zahl der Laserpulse, die besonders weit entfernt von ihrem Soll-Auftreffort auftreffen, verringert sich. Die Ablation wird dadurch insgesamt präziser.

Die in Schritt b) zu ermittelnde Beziehung kann beispielsweise so aussehen, dass an dem Patienten sorgfältige Messungen vorab durchgeführt werden, bei denen die Beleuchtung variiert wird. Dadurch ändert sich der Durchmesser der Pupille. Man misst die beiden Koordinaten des Mittelpunkts der Pupille relativ zum Mittelpunkt des Limbus und erhält so eine Tabelle, aus der in Schritt d) die Referenzkoordinaten entnommen werden können.

Bevorzugt wird jedoch eine Formel verwendet. Das Ermitteln der Beziehung in Schritt b) umfasst dann, dass vier Eichkoordinaten verwendet werden, von denen zumindest zwei Eichkoordinaten neu gemessen werden, die den Abstand des Mittelpunkts der beleuchteten Pupille relativ zum Mittelpunkt des Limbus bei unverdrehtem Auge angeben. Bevorzugt wird hierbei die so genannte photopische Pupille erzeugt. Dies ist die Pupille im Zustand minimalen Durchmessers, der sich bei besonders hellem Licht einstellt. Sind die Zentrumskoordinaten die Koordinaten des Mittelpunkts der scotopischen Pupille, können die Zentrumskoordinaten dann die beiden weiteren Eichkoordinaten sein. Sind die Zentrumskoordinaten die Koordinaten eines Ablationszentrums, das nicht die scotopische Pupille ist, sollten als zwei weitere Eichkoordinaten diejenigen gemessen werden, die den Abstand des Mittelpunkts der scotopischen Pupille relativ zum Mittelpunkt des Limbus angeben. Aus allen Eichkoordinaten wird dann eine Formel abgeleitet. Theoretisch ist die Einbeziehung von Extrapolation möglich, bevorzugt handelt es sich bei der Formel jedoch um eine reine Interpolationsformel, was durch Aufnahme der Eichkoordinaten bei sowohl der scotopischen als auch der photopischen Pupille ja möglich ist. Grundsätzlich kann linear interpoliert werden. Hierbei wird davon ausgegangen, dass der Mittelpunkt der Pupille längs einer geraden Strecke wandert, deren Endpunkte der Mittelpunkt der scotopischen und der Mittelpunkt der photopischen Pupille sind.

Das erfindungsgemäße Verfahren dient ja bevorzugt dazu, den Effekt einer Verdrehung zu berücksichtigen. Die Verdrehung ist zum Beispiel mit einer Winkelgröße quantifizierbar. Zwar ist es nicht notwendig, dass diese Winkelgröße explizit berechnet wird. Es ist aber bevorzugt vorgesehen, dass das in Vorgang f) genannte Berechnen umfasst, dass eine verwendete Formel so gewählt ist, dass anhand der gemessenen Ist-Koordinaten und der ermittelten Referenzkoordinaten auf die Verdrehung des Auges zurückgeschlossen ist und in der Ableitung der Formel eine diesbezügliche Winkelgröße verwendet ist. Bei der Ableitung der Formel soll eine diesbezügliche Winkelgröße zu einer dem scotopischen unverdrehten Auge zugeordneten Winkelgröße hinzugezählt sein, und das Ergebnis dieser Hinzuzählung soll unter Berücksichtigung der Auftrefffortskoordinaten in diese Formel eingegangen sein.

Mit anderen Worten bedeutet dies, dass die Formel so gewählt ist, dass ein Verdrehwinkel der nicht-scotopischen Pupille, nämlich der Pupille bei der Beleuchtung, die für die Behandlung vorgegeben ist, so behandelt wird, als handle es sich um den Verdrehwinkel des Ablationszentrums, d. h. der scotopischen Pupille. Dies stellt eine vernünftige Vorgehensweise dar.

Nachfolgend wird eine bevorzugte Ausführungsform der Erfindung unter Bezug auf die Zeichnungen beschrieben, wobei

Fig. 1    schematisch den Limbus, die scotopische und die photopische Pupille und deren jeweiligen Mittelpunkt veranschaulicht,

Fig. 2    schematisch die Koordinatenpunkte bei unterschiedlichen Messungen bzw. Berechnungen relativ zum Mittelpunkt des Limbus veranschaulicht.

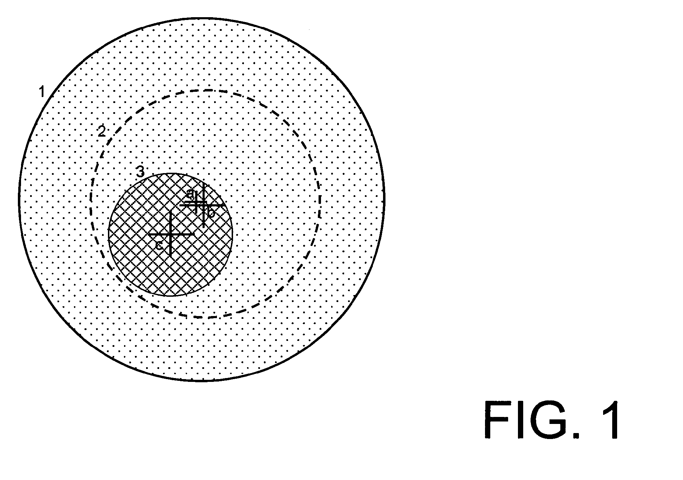

Mithilfe von Eyetrackern ist es gut möglich, die Kontur des Limbus zu erfassen, der in Fig. 1 mit 1 gekennzeichnet ist. Während einer laufenden Behandlung kann anhand dieser Kontur auch der Mittelpunkt des Limbus ermittelt werden, der in Fig. 1 mit a bezeichnet ist.

Genauso wie der Limbus erfasst werden kann, kann auch die Kontur der Pupille erfasst werden. In Fig. 1 mit 2 bezeichnet ist die Kontur der scotopischen Pupille, d. h. der Pupille im Zustand ihrer maximalen Öffnung. Auch ihr Mittelpunkt b kann ermittelt werden. Der Mittelpunkt b der scotopischen Pupille 2 fällt in fast aller Regel nicht mit dem Mittelpunkt a des Limbus 1 zusammen.

Zieht sich die Pupille zusammen, ändert sich auch ihr Mittelpunkt. Fig. 1 zeigt zusätzlich zur Kontur der scotopischen Pupille die Kontur der photopischen Pupille, d. h. die Kontur der Pupille bei ihrer minimalen Ausdehnung. Der Mittelpunkt c der photopischen Pupille fällt in aller Regel weder mit dem Mittelpunkt b der scotopischen Pupille zusammen, noch mit dem Mittelpunkt a des Limbus. Die Abweichung ist in Fig. 1 in stark übertriebener Weise dargestellt.

Vor einer Ablation wird ein Ablationsprofil definiert, und damit dieses Profil bei der Ablation erzeugt wird, werden eine Vielzahl von Ablationsorten definiert, und zu jedem Ablationsort wird festgelegt, wie viele Laserpulse auf diesen auftreffen sollen. Der Ablationsort wird üblicherweise relativ zum Mittelpunkt b der scotopischen Pupille definiert. Die Ablationsorte werden durch zwei Koordinaten $\Delta x_{Abl-AblC}$ und $\Delta y_{Abl-AblC}$ relativ zum Ablationszentrum (AbIC) definiert, welches seinerseits die Relativkoordinaten $\Delta x_{AbIC-L}$ und $\Delta y_{ablC-L}$ hat.

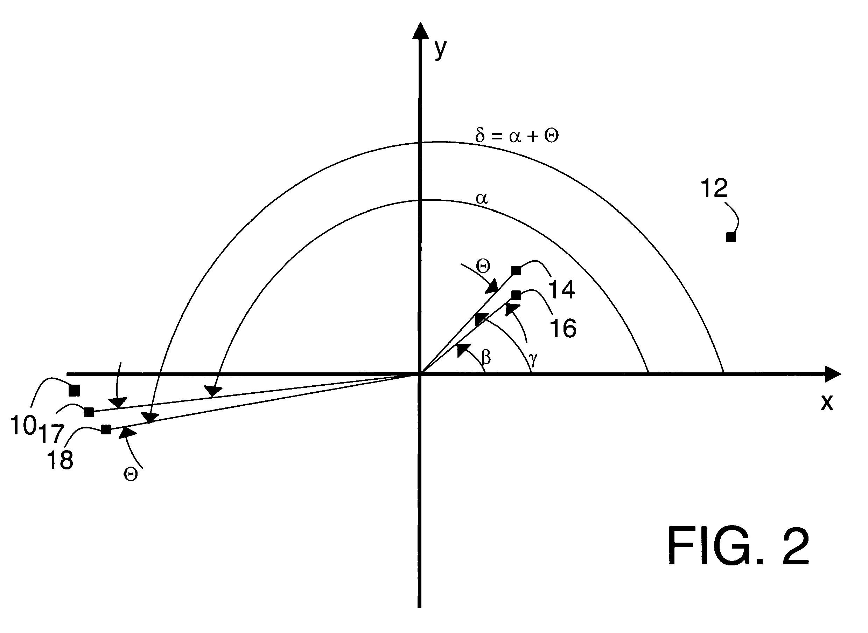

Da während der Ablation eine Beleuchtung bedingt, dass die Pupille nicht scotopisch ist, ist der Mittelpunkt b nicht laufend ermittelbar. Laufend ermittelbar ist der Mittelpunkt a des Limbus. Vorab werden daher als Eichkoordinaten die Koordinaten des Mittelpunkts b der scotopischen Pupille relativ zum Mittelpunkt a des Limbus gemessen. Fig. 2 zeigt den Punkt 10, der beispielhaft die Koordinaten $\Delta x_{Sc-L}$ und $\Delta y_{Sc-L}$ habe. Bei der Bezeichnung steht "Sc" für scotopisch und "L" für Limbus. Die Nennung beider Größen im Index soll verdeutlichen, dass es sich um eine auf den Mittelpunkt a des Limbus bezogene Relativgröße handelt. Hier wird davon ausgegangen, dass Punkt 10 auch gleichzeitig das Ablationszentrum mit den Zentrumskoordinaten $\Delta x_{AbIC-L}$ und $\Delta y_{AbIC-L}$ angibt. Die nachfolgenden Formeln sind nicht auf diesen Fall beschränkt.

Im Rahmen des vorliegenden Verfahrens wird nun zusätzlich für die photopische Pupille deren Mittelpunkt c relativ zum Mittelpunkt a des Limbus gemessen, und man erhält zum Beispiel den Punkt 12 im Koordinatensystem aus Fig. 2, der die Koordinaten $\Delta x_{Ph-L}$ und $\Delta y_{Ph-L}$ habe, wobei "Ph" für photopisch steht und abermals klargestellt sein soll, dass es sich um eine Relativgröße bezogen auf den Mittelpunkt a des Limbus handelt. Zusätzlich zu den Koordinaten der Punkte 10 und 12 wird der Durchmesser der Pupille gemessen, und zwar der Durchmesser ScS für die scotopische Pupille und der Durchmesser PhS für die photopische Pupille (wobei das letzte "S" jeweils für "Size", Größe, stehen soll).

Im Folgenden wird nun von der Vorstellung ausgegangen, dass sich der Mittelpunkt der Pupille mittleren Durchmessers genau auf einer geraden Strecke zwischen dem Mittelpunkt b des scotopischen Pupille und dem Mittelpunkt c der photopischen Pupille befindet. Bezogen auf den Mittelpunkts a des Limbus liegen dessen Koordinaten dann auf einer die Punkte 10 und 12 aus Fig. 2 verbindenden geraden Strecke. Es wird nun die Pupillengröße PS (für "Pupil Size") der Pupille unter den bei der Behandlung vorherrschenden Beleuchtungsbedingungen gemessen und eine zu erwartende Stellung des Mittelpunkts der Pupille mit der Größe PS relativ zum Limbus rechnerisch abgeleitet. Bei linearer

Interpolation erhält man:

$$\Delta x_{RP-L}(PS) = \Delta x_{Ph-L} + \frac{(\Delta x_{Sc-L} - \Delta x_{Ph-L})(PS - PhS)}{ScS - PhS}$$

$$\Delta y_{RP-L}(PS) = \Delta y_{Ph-L} + \frac{(\Delta y_{Sc-L} - \Delta y_{Ph-L})(PS - PhS)}{ScS - PhS}$$

wobei das "R" für Referenzkoordinate stehen soll und die in Klammern gesetzten "PS" verdeutlichen, dass es sich bei den hier zu berechnenden Größen um von der Pupillengröße abhängige Größen handelt.

Die beiden Formeln drücken eine lineare Interpolation mit einer Interpolationsgeraden aus, die durch die Punkte 10 und 12 verläuft. Dies erkennt man daran, dass, wenn man für PS = PhS setzt, dann genau die Koordinaten des Punktes 12 erhält, und wenn man für PS = ScS einsetzt, genau die Koordinaten des Punktes 10 erhält. Die oben genannten Koordinaten $\Delta x_{RP-L}(PS)$ und $\Delta y_{RP-L}(PS)$ seien die Koordinaten des in Fig. 2 eingezeichneten Punktes 14.

Wie gesagt, ist es auch möglich, mithilfe eines Eyetrackers und Mustererkennung den Mittelpunkt der Pupille mit der Größe PS, also unter der bei der Behandlung vorherrschenden Beleuchtung, zu messen ebenso wie auch den Mittelpunkt des Limbus und somit die Ist-Koordinaten des Mittelpunkts der Pupille relativ zum Mittelpunkt des Limbus zu erfassen. Vorliegend seien diese durch den Punkt 16 in Fig. angegeben, der die Koordinaten $\Delta x_{MP-L}$ und $\Delta y_{MP-L}$ habe, wobei "M" für Messwert steht. Verbindet man nun den Punkt 14 mit dem Ursprung des Koordinatensystems in Fig. 2, so lässt sich ein Winkel γ dieser Verbindung zur X-Achse definieren. Hierbei gilt:

$$\gamma = \arctan\left(\frac{\Delta y_{RP-L}(PS)}{\Delta x_{RP-L}(PS)}\right)$$

Genauso lässt sich zwischen einer Verbindung des Punkts 16 mit dem Ursprung und der x-Achse ein Winkel β definieren, für den gilt:

$$\beta = \arctan\left(\frac{\Delta y_{MP-L}}{\Delta x_{MP-L}}\right)$$

Die Differenz dieser Winkel lässt sich als Winkel Θ definieren, wobei gilt:

$$\Theta = \gamma - \beta = \arctan\left(\frac{\dfrac{\Delta y_{MP-L}}{\Delta x_{MP-L}} - \dfrac{\Delta y_{RP-L}(PS)}{\Delta x_{RP-L}(PS)}}{1 + \dfrac{\Delta y_{MP-L}}{\Delta x_{MP-L}}\dfrac{\Delta y_{RP-L}(PS)}{\Delta x_{RP-L}(PS)}}\right)$$

Da der Punkt 14 die erwarteten Koordinaten bei unverdrehtem Auge angibt und der Punkt 16 die Ist-Koordinaten angibt, wird vorliegend davon ausgegangen, dass der Winkel Θ genau den Effekt einer Verdrehung des Auges während der Behandlung widerspiegelt. Soll nun ein Laserpuls zum Zwecke der Ablation von Hornhaut zielgenau auf einen Soll-Auftreffort geleitet werden, muss diese Verdrehung berücksichtigt werden. Die einfachste Möglichkeit besteht darin, davon auszugehen, dass sich auch der Zielort um genau denselben Winkel Θ verdreht. Ist der Zielort durch den Punkt 17 angegeben, der die relativ auf den Mittelpunkt des Limbus bezogenen Koordinaten $\Delta x_{Abl-L}$ und $\Delta y_{Abl-L}$ hat, dann gilt $\Delta x_{Abl-L} = \Delta x_{AblC-L} + \Delta x_{Abl-AblC}$ und $\Delta y_{Abl-L} = \Delta y_{AblC-L} + \Delta y_{Abl-AblC}$. Ist nun α der Winkel von einer Verbindung zwischen dem Punkt 17 und dem Ursprung des Koordinatensystems zur positiven X-Achse, wobei also gilt:

$$\alpha = \arctan\left(\frac{\Delta y_{Abl-L}}{\Delta x_{Abl-L}}\right) = \arctan\left(\frac{\Delta y_{AblC-L} + \Delta y_{Abl-AblC}}{\Delta x_{AblC-L} + \Delta y_{Abl-AblC}}\right)$$

dann muss dieser Winkel $\alpha$ um die Verdrehung korrigiert werden zu:

$$\delta = \alpha + \Theta$$

Man erhält dann den Punkt 18. Relativ zu diesem Punkt 18 ist nun bei vorgegebenen Auftreffortskoordinaten $\Delta x_{Abl-AblC}$ und $\Delta y_{Abl-AblC}$ der Soll-Auftreffort neu zu berechnen. Zweckmäßigerweise berechnet man diesen direkt relativ zum Mittelpunkt des Limbus, man berechnet also die Positionskoordinaten $\Delta x_{Abl-L,korr}$ und $\Delta y_{Abl-L,korr}$. Da sich der Soll-Auftreffpunkt beim Verdrehen des Auges mitgedreht hat, erhält man bei gleichbleibendem Vektorbetrag für diese Größen:

$$\Delta x_{Abl-L,korr} = \left(\left(\Delta x_{AblC-L} + \Delta x_{Abl-AblC}\right)^2 + \left(\Delta y_{Sc-L} + \Delta y_{Abl-AblC}\right)^2\right)^{1/2} \cos\delta$$

$$\Delta y_{Abl-L,korr} = \left(\left(\Delta x_{AblC-L} + \Delta x_{Abl-AblC}\right)^2 + \left(\Delta y_{Sc-L} + \Delta y_{Abl-AblC}\right)^2\right)^{1/2} \sin\delta$$

Es lässt sich nach aufwändigen Zwischenschritten, bei denen aber lediglich trigonometrische Beziehungen verwendet werden, zum Einsetzen in diese Formeln für $\cos\delta$ und $\sin\delta$ ableiten:

$$\cos\delta = \frac{\left(\Delta x_{MP-L}\Delta x_{RP-L}(PS) + \Delta y_{MP-L}\Delta y_{RP-L}(PS)\right)\Delta x_{AblC-L}}{\left(\left(\Delta x_{AblC-L}^2 + \Delta y_{AblC-L}^2\right)\left(\Delta x_{MP-L}^2 + \Delta y_{MP-L}^2\right)\left(\Delta x_{RP-L}^2(PS) + \Delta y_{RP-L}^2(PS)\right)\right)^{1/2}}$$
$$+ \frac{\left(\Delta x_{MP-L}\Delta y_{RP-L}(PS) - \Delta y_{MP-L}\Delta x_{RP-L}(PS)\right)\Delta y_{AblC-L}}{\left(\left(\Delta x_{AblC-L}^2 + \Delta y_{AblC-L}^2\right)\left(\Delta x_{MP-L}^2 + \Delta y_{MP-L}^2\right)\left(\Delta x_{RP-L}^2(PS) + \Delta y_{RP-L}^2(PS)\right)\right)^{1/2}}$$

$$\sin\delta = \frac{\left(\Delta y_{MP-L}\Delta x_{RP-L}(PS) - \Delta x_{MP-L}\Delta y_{RP-L}(PS)\right)\Delta x_{AblC-L}}{\left(\left(\Delta x_{AblC-L}^2 + \Delta y_{AblC-L}^2\right)\left(\Delta x_{MP-L}^2 + \Delta y_{MP-L}^2\right)\left(\Delta x_{RP-L}^2(PS) + \Delta y_{RP-L}^2(PS)\right)\right)^{1/2}}$$
$$+ \frac{\left(\Delta x_{MP-L}\Delta x_{RP-L}(PS) + \Delta y_{MP-L}\Delta y_{RP-L}(PS)\right)\Delta y_{AblC-L}}{\left(\left(\Delta x_{AblC-L}^2 + \Delta y_{AblC-L}^2\right)\left(\Delta x_{MP-L}^2 + \Delta y_{MP-L}^2\right)\left(\Delta x_{RP-L}^2(PS) + \Delta y_{RP-L}^2(PS)\right)\right)^{1/2}}$$

Nach diesem Einsetzen ergeben sich somit die Positionskoordinaten $\Delta x_{Abl-L,korr}$ und $\Delta y_{Abl-L,korr}$, welche ja auf den Mittelpunkt des Limbus bezogen sind, anhand von acht verschiedenen Größen, nämlich des Paares der Auftreffortskoordinaten $\Delta x_{Abl-AblC}$ und $\Delta y_{Abl-AblC}$, der gemessenen beiden Zentrumskoordinaten $\Delta x_{AblC-L}$ und $\Delta y_{AblC-L}$, den zuletzt gemessenen beiden Ist-Koordinaten $\Delta x_{MP-L}$ und $\Delta y_{MP-L}$ und den ermittelten beiden Referenzkoordinaten $\Delta x_{RP-L}(PS)$ und $\Delta y_{RP-L}(PS)$.
Beim Ermitteln der Formel wurde auf die Verdrehung des Auges zurückgeschlossen und eine diesbezügliche Winkelgröße $\Theta$ zu einer dem scotopischen unverdrehten Auge zugeordneten Winkelgröße $\alpha$ hinzugezählt. Anhand des

Ergebnisses dieser Hinzuzählung wurde unter Berücksichtigung der Auftefffortskoordinaten die gesamte Formel abgeleitet.

Kurz zusammengefasst: Die Koordinaten der Punkte 10 und 12 werden vorab gemessen. Auch die Pupillengröße PS wird vorab gemessen. Bei laufender Laserbehandlung, bei der die Laserpulse gegebenenfalls mit einer Frequenz von z. B. 200 Hz abgegeben wird, kann kurzfristig der Mittelpunkt der Pupille und der Mittelpunkt des Limbus und somit der Relativpunkt 16 gemessen werden. Die Steuerung des Lasers funktioniert ohnehin so, dass die Soll-Auftrefforte nacheinander durchlaufen werden, und anhand der schnell gemessenen Koordinaten des Punktes 16 kann dann mithilfe der übrigen sechs, vorab bestimmten Größen sehr kurzfristig die Verdrehung des Auges rechnerisch einbezogen werden. Die Positionskoordinaten, auf den Limbus bezogen, sind dann berechenbar, und da die Position des Mittelpunkts des Limbus während der Behandlung wie üblich relativ zu einem Bezugssystem einer Zieleinrichtung gemessen wird, kann die Zieleinrichtung dann die Laserpulse zu dem durch die Positionskoordinaten angegebenen Ort leiten, d. h. zu dem Ort, der aufgrund einer Berücksichtigung der Verdrehung berechnet ist. Der Messpunkt 16 kann sich sehr kurzfristig ändern, weil das Auge während der Behandlung schnell verdreht werden kann. Diese Änderung kann aber ebenso auch kurzfristig erfasst werden. Die Verdrehung wird dadurch wesentlich schneller berücksichtigt als im Stande der Technik.

**Patentansprüche**

1.  Verfahren zum Steuern des Auftreffortes von Laserpulsen bei einer Ablation von Hornhaut an einem Auge, mit den Schritten:

    a) Festlegen oder Messen von zwei Zentrumskoordinaten ($\Delta x_{AblC-L}$, $\Delta y_{AblC-L}$), die den Abstand eines Ablationszentrums relativ zum Mittelpunkt (a) des Limbus des Auges bei unverdrehtem Auge angeben,
    b) Ermitteln einer Beziehung zwischen dem Durchmesser (PS) der Pupille und dem durch zwei Koordinaten ($\Delta x_{RP-L}(PS)$, $\Delta y_{RP-L}(PS)$) angegebenen Abstand des Mittelpunkts der Pupille relativ zum Mittelpunkt (a) des Limbus bei unverdrehtem Auge,
    c) Festlegen zweier Auftreffortskoordinaten ($\Delta x_{Abl-AblC}$, $\Delta y_{Abl-AblC}$) des Soll-Auftreffortes für eine Vielzahl von Laserpulsen relativ zum Ablationszentrum,
    d) Messen des Durchmessers der Pupille bei für die Behandlung vorgegebener Beleuchtung und Zuordnen zweier Koordinaten aufgrund der ermittelten Beziehung als Referenzkoordinaten ($\Delta x_{RP-L}(PS)$, $\Delta x_{RP-L}(PS)$) und Wiederholen folgender Vorgänge bei gleichbleibender Beleuchtung:
    e) Messen der zwei Ist-Koordinaten ($\Delta x_{MP-L}$, $\Delta y_{MP-L}$), die den Abstand des Mittelpunkts der Pupille zum Mittelpunkt (a) des Limbus angeben,
    f) Berechnen zweier auf den Mittelpunkt des Limbus bezogener Positionskoordinaten ($\Delta x_{Abl-L,korr}$, $\Delta y_{Abl-L,korr}$) anhand von:

        - jeweils eines ausgewählten Paares von Auftreffortskoordinaten ($\Delta x_{Abl-AblC}$, $\Delta y_{Abl-AblC}$),
        - den beiden Zentrumskoordinaten ($\Delta x_{AblC-L}$, $\Delta y_{AblC-L}$),
        - den zuletzt gemessenen beiden Ist-Koordinaten ($\Delta x_{MP-L}$, $\Delta y_{MP-L}$), und
        - den ermittelten beiden Referenzkoordinaten ($\Delta x_{RP-L}(PS)$, $\Delta y_{RP-L}(PS)$),

    g) Messen der Position des Mittelpunkts (a) des Limbus relativ zu einem Bezugssystem einer Zieleinrichtung für Laserpulse,
    h) Umrechnen der Positionskoordinaten auf das Bezugssystem der Zieleinrichtung,
    i) Einstellen der Zieleinrichtung derart, dass abgegebene Laserpulse zu dem durch die Positionskoordinaten angegebenen Ort geleitet werden.

2.  Verfahren nach Anspruch 1,
    **dadurch gekennzeichnet,**
    **dass** das Ermitteln der Beziehung umfasst, dass vier Eichkoordinaten ($\Delta x_{Sc-L}$, $\Delta y_{Sc-L}$; $\Delta x_{Ph-L}$, $\Delta y_{Ph-L}$) bereitgestellt werden, von denen zumindest zwei Eichkoordinaten ($\Delta x_{Ph-L}$, $\Delta y_{Ph-L}$) gemessen werden, die den Abstand des Mittelpunkts (c) der beleuchteten, und vorzugsweise der photopischen Pupille relativ zum Mittelpunkt (a) des Limbus bei unverdrehtem Auge angeben, und dass aus allen Eichkoordinaten eine Formel, bevorzugt eine Interpolationsformel, abgeleitet wird.

3.  Verfahren nach Anspruch 1 oder 2,
    **dadurch gekennzeichnet,**
    **dass** das Berechnen in Vorgang f) umfasst, dass eine verwendete Formel so gewählt ist, dass anhand der gemessenen Ist-Koordinaten und der ermittelten Referenzkoordinaten auf die Verdrehung des Auges zurückgeschlossen ist, eine diesbezügliche Winkelgröße ($\Theta$) zu einer dem scotopischen unverdrehten Auge zugeordneten Winkelgröße ($\alpha$) hinzugezählt ist und das Ergebnis ($\delta$) dieser Hinzuzählung unter Berücksichtigung der Auftreffortskoordinaten ($\Delta x_{Abl-AblC}$, $\Delta y_{Abl-AblC}$) in diese Formel eingegangen ist.

**Geänderte Patentansprüche gemäss Regel 137(2) EPÜ.**

**1.** Verfahren zum Steuern des Auftreffortes von Laserpulsen bei einer Ablation von Hornhaut an einem Auge, mit den Schritten:

a) Festlegen oder Messen von zwei Zentrumskoordinaten ($\Delta x_{AblC-L}$, $\Delta y_{AblC-L}$), die den Abstand eines Ablationszentrums relativ zum Mittelpunkt (a) des Limbus des Auges bei unverdrehtem Auge angeben,
b) Ermitteln einer Beziehung zwischen dem Durchmesser (PS) der Pupille und dem durch zwei Koordinaten ($\Delta x_{RP-L}$ (PS), $\Delta y_{RP-L}$(PS)) angegebenen Abstand des Mittelpunkts der Pupille relativ zum Mittelpunkt (a) des Limbus bei unverdrehtem Auge,
c) Festlegen zweier Auftreffortskoordinaten ($\Delta x_{Abl-AblC}$, $\Delta y_{Abl-AblC}$) des Soll-Auftreffortes für eine Vielzahl von Laserpulsen relativ zum Ablationszentrum,
d) Messen des Durchmessers der Pupille bei für die Behandlung vorgegebener Beleuchtung und Zuordnen zweier Koordinaten aufgrund der ermittelten Beziehung als Referenzkoordinaten ($\triangle x_{RP-L}$ (PS), $\triangle x_{RP-L}$ (PS))

und Durchführen folgender Vorgänge bei gleichbleibender Beleuchtung:

e) Messen der zwei Ist-Koordinaten ($\Delta x_{MP-L}$, $\Delta y_{MP-L}$), die den Abstand des Mittelpunkts der Pupille zum Mittelpunkt (a) des Limbus angeben,
f) Berechnen zweier auf den Mittelpunkt des Limbus bezogener Positionskoordinaten ($\Delta x_{Abl-L,korr}$, $\Delta y_{Abl-L,korr}$) anhand von:

- jeweils eines ausgewählten Paares von Auftreffortskoordinaten ($\Delta x_{Abl-AblC}$, $\Delta y_{Abl-AblC}$),
- den beiden Zentrumskoordinaten ($\Delta x_{AblC-L}$, $\Delta y_{AblC-L}$),
- den zuletzt gemessenen beiden Ist-Koordinaten ($\Delta x_{MP-L}$, $\Delta y_{MP-L}$), und
- den ermittelten beiden Referenzkoordinaten ($\Delta x_{RP-L}$(PS), $\Delta y_{RP-L}$(PS)),

g) Messen der Position des Mittelpunkts (a) des Limbus relativ zu einem Bezugssystem einer Zieleinrichtung für Laserpulse,
h) Umrechnen der Positionskoordinaten auf das Bezugssystem der Zieleinrichtung,
i) Einstellen der Zieleinrichtung derart, dass abgegebene Laserpulse zu dem durch die Positionskoordinaten angegebenen Ort geleitet werden.

**2.** Verfahren nach Anspruch 1,
**dadurch gekennzeichnet,**
**dass** das Ermitteln der Beziehung umfasst, dass vier Eichkoordinaten ($\Delta x_{Sc-L}$, $\Delta y_{Sc-L}$; $\Delta x_{Ph-L}$, $\Delta y_{Ph-L}$) bereitgestellt werden, von denen zumindest zwei Eichkoordinaten ($\Delta x_{Ph-L}$, $\Delta y_{Ph-L}$) gemessen werden, die den Abstand des Mittelpunkts (c) der beleuchteten, und vorzugsweise der photopischen Pupille relativ zum Mittelpunkt (a) des Limbus bei unverdrehtem Auge angeben, und dass aus allen Eichkoordinaten eine Formel, bevorzugt eine Interpolationsformel, abgeleitet wird.

**3.** Verfahren nach Anspruch 1 oder 2,
**dadurch gekennzeichnet,**
**dass** das Berechnen in Vorgang f) umfasst, dass eine verwendete Formel so gewählt ist, dass anhand der gemessenen Ist-Koordinaten und der ermittelten Referenzkoordinaten auf die Verdrehung des Auges zurückgeschlossen ist, eine diesbezügliche Winkelgröße ($\Theta$) zu einer dem scotopischen unverdrehten Auge zugeordneten Winkelgröße ($\alpha$) hinzugezählt ist und das Ergebnis ($\delta$) dieser Hinzuzählung unter Berücksichtigung der Auftreffortskoordinaten ($\Delta x_{Abl-AblC}$, $\Delta y_{Abl-AblC}$) in diese Formel eingegangen ist.

FIG. 1

FIG. 2

**Europäisches Patentamt**

**ERKLÄRUNG**

die nach Regel 45 des Europäischen Patentübereinkommens für das weitere Verfahren als
europäischer Recherchenbericht gilt

Nummer der Anmeldung

EP 06 02 6333

| Die Recherchenabteilung ist der Auffassung, daß die vorliegende Patentanmeldung den Vorschriften des EPÜ in einem solchen Umfang nicht entspricht, daß sinnvolle Ermittlungen über den Stand der Technik auf der Grundlage aller Patentansprüche nicht möglich sind. | KLASSIFIKATION DER ANMELDUNG (IPC) |
|---|---|
| Grund: | INV. A61F9/01 |

Eine sinnvolle Recherche auf der Grundlage aller Ansprüche ist nicht möglich, da sich diese auf ein Verfahren zur chirurgischen Behandlung des menschlichen oder tierischen Körpers beziehen - Artikel 52 (4) EPÜ -
Das Verfahren zum Steuern des Auftreffortes von Laserpulsen bei einer Ablation von Hornhaut an einem Auge, beinhaltend das Wiederholen der Vorgänge e) - i), wie in Anspruch 1 definiert, wird als eine Methode zur chirurgischen Behandlung des menschlichen oder tierischen Körpers angesehen. In der Beschreibung, wird auf S. 4, Z. 20-21 explizit darauf hingewiesen, dass es "naturgemäß zu dem Verfahren gehört, dass die Wiederholungen der einzelnen Vorgänge f) bis i) jeweils mit der Abgabe mindestens eines Laserpulses einhergehen". Dieses Abgeben von Laserpulsen auf die Hornhaut eines Auges greift in die Struktur des Organismus ein und ist daher von chirurgischer Natur. Eine solche Methode ist nach Artikel 52(4) EPC von der Patentierbarkeit ausgeschlossen.
Folglich müssen Ansprüche 1-3 gestrichen werden. Die entsprechenden Passagen der Beschreibung müssen als ausschließlich beschreibend kenntlich gemacht werden.
Es wird ferner darauf hingewiesen, dass der als chirurgisch angesehene Schritt des Abgebens von mindestens einem Laserpuls auf die Hornhaut eines Auges weiterhin als essentiell und damit im weiteren Verfahren als nicht streichbar angesehen wird: Das in Anspruch 1 der vorliegenden Anmeldung beschriebene Wiederholen der Vorgänge e) bis i) beschreibt das typische Vorgehen
-/--

| Recherchenort | Abschlußdatum | Prüfer |
|---|---|---|
| München | 4. April 2007 | Jansen, Birte |

**Europäisches Patentamt**

**ERKLÄRUNG**

die nach Regel 45 des Europäischen Patentübereinkommens für das weitere Verfahren als
europäischer Recherchenbericht gilt

Nummer der Anmeldung

EP 06 02 6333

| Die Recherchenabteilung ist der Auffassung, daß die vorliegende Patentanmeldung den Vorschriften des EPÜ in einem solchen Umfang nicht entspricht, daß sinnvolle Ermittlungen über den Stand der Technik auf der Grundlage aller Patentansprüche nicht möglich sind. | **KLASSIFIKATION DER ANMELDUNG (IPC)** |
|---|---|

Grund:

bei der Verwendung eines
"Eye-tracking"-Systems in der refraktiven
Hornhautchirurgie. Dabei wird auf der
Basis der aktuell erfassten Augen-Position
die für die nächste Laserschussabgabe
notwendige Position ermittelt. Ein
Herausstreichen der Laserschussabgabe
würde die Methode ad absurdum führen, da
die ermittelte Laserschuss-Position mit
der aktuellen Augenposition korreliert ist
und bei nicht fixiertem Auge zu einem
späteren Zeitpunkt des Verfahrens keine
Gültigkeit mehr besitzt.

Der Anmelder wird darauf hingewiesen, dass
im Zuge der Prüfung eine Recherche
durchgeführt werden kann, sollten die
einer Erklärung gemäss Regel 45 EPÜ
zugrundeliegenden Mängel behoben worden
sein (Vgl. EPA-Richtlinien C-VI, 8.5).
-----

| Recherchenort | Abschlußdatum | Prüfer |
|---|---|---|
| München | 4. April 2007 | Jansen, Birte |

EPO FORM 1504 (P04C39)